# EUROPÄISCHE PATENTSCHRIFT

(11) **EP 2 564 764 B1**
(45) Veröffentlichungstag und Bekanntmachung des Hinweises auf die Patenterteilung: **10.08.2016**
(21) Anmeldenummer: 12180837.2
(22) Anmeldetag: 17.08.2012
(51) Int. Cl.: A61B 3/107, A61B 3/10

(54) **Ophthalmologisches Analysegerät und Verfahren**
Ophthalmological analysis apparatus and method
Appareil d'analyse ophtalmologique et procédé

(30) Priorität: 30.08.2011 DE 102011081827
(43) Veröffentlichungstag der Anmeldung: 06.03.2013
(73) Patentinhaber: Oculus Optikgeräte GmbH, 35582 Wetzlar-Dutenhofen (DE)
(72) Erfinder: Steinmüller Andreas, 35435 Wettenberg (DE)
(74) Vertreter: advotec.

(56) Entgegenhaltungen:
- EP-A1- 1 723 900
- EP-A1- 2 016 888
- EP-A2- 0 697 611
- EP-A2- 1 857 043
- DE-A1-102004 055 683
- US-A- 5 159 361
- US-A1- 2008 309 872
- US-A1- 2009 046 250

## Beschreibung

Die Erfindung betrifft ein ophthalmologisches Analysegerät mit den Merkmalen des Oberbegriffs des Anspruchs 1 sowie ein Analyseverfahren mit den Merkmalen des Oberbegriffs des Anspruchs 5.

Bei den aus dem Stand der Technik bekannten Analysegeräten bzw. Topografiesystemen wird regelmäßig ein Auge mit monochromatischen, wie beispielsweise infrarotem Licht beleuchtet, um eine Blendung der zu untersuchenden Person bzw. des Probanden weitestgehend zu vermeiden. So sind Topografiesysteme bekannt, die neben einer Messung einer Oberfläche eines Auges, auch pupillometrische Messungen ermöglichen. Insbesondere bei einer Beleuchtung des Auges mit monochromatischen, nur bedingt sichtbaren Licht wird eine Kontraktion der Pupille wirkungsvoll vermieden, sodass sich eine derartige Beleuchtung besonders gut für pupillometrische Messungen eignet. Topografiesysteme können aber auch über mehrere Lichtfarben zur Beleuchtung eines Auges verfügen. Dann werden zum Beispiel Placidoringe in unterschiedlichen, monochromatischen Lichtfarben auf das Auge projiziert. Dies dient im Wesentlichen zur Unterscheidung der Ringe des auf dem Auge abgebildeten Bildmusters und alleine zum Zwecke einer Topographiebestimmung. Derartige Placidoringe in jeweils unterschiedlichen, monochromatischen Lichtfarben bilden folglich alleine die erste Lichtquelle aus. Weiter ist es bekannt, eine Untersuchung von Meibomdrüsen unter infrarotem Licht durchzuführen.

Auch können Analysegeräte zur Messung einer Topografie bzw. sogenannte Keratometer zur nicht invasiven Analyse eines Tränenfilms auf einem Auge verwendet werden. Dabei wird das auf die Oberfläche des Auges projizierte Bildmuster im Wesentlichen kontinuierlich aufgenommen, wobei ein Aufreißen des Tränenfilms durch eine Veränderung des Bildmusters erkannt werden kann. Um eine Aussage über eine Qualität des Tränenfilms treffen zu können, wird regelmäßig so eine Aufreißzeit desselben gemessen. Diese Messung wird ebenfalls mittels einer Beleuchtung des Auges mit infrarotem Licht durchgeführt. Beispielsweise ist eine Messung eines Tränenfilms, neben der Topografie der Oberfläche des Auges, bei einer Auswahl von Kontaktlinsen von großer Bedeutung. Weiter ist eine Analyse eines Tränenfilms alleine auf eine Verteilung des Tränenfilms über das Auge beschränkt. Nachteilig bei dem bekannten Analysegerät bzw. Verfahren ist, dass die Möglichkeiten zur Untersuchung eines Auges begrenzt sind. Es ist daher wünschenswert, die Untersuchungsmöglichkeiten eines derartigen Gerätes zu erweitern, um gegebenenfalls weitere und detaillierte Messergebnisse zu erhalten, die beispielsweise für eine verbesserte Kontaktlinsenauswahl und Anpassung nutzbar sind.

1 723 900 offenbart eine Vorrichtung und ein Verfahren zur Auswertung des trockenen Zustands des Auges mittels Messung der Bewegungsgeschwindigkeit der Tränenflüssigkeit.

Die DE 10 2004 055 683 A1 beschreibt ein Operationsmikroskop bei dem insbesondere ist in einem Strahlengang des Mikroskops eine Projektionsvorrichtung angeordnet ist, mittels der ein aus Markierungen bestehendes Bild in den Strahlengang so eingekoppelt werden kann, dass das einem Betrachter sichtbare Bild eines zu operierenden Auges von dem eingekoppelten Bild überlagert wird. Weiter ist eine Vergrößerung des Bildes des Auges über ein sogenanntes Zoomsystem veränderbar.

Die EP 0 697 611 A2 betrifft ein Mikroskop für Augenoperationen, welches mit einem System zur optischen Kohärenztomographie kombiniert ist. Insbesondere ist eine Projektionsvorrichtung zur Beleuchtung des zu operierenden Auges vorgesehen und das Mikroskop verfügt über einen Vergrößerungswechsler, mit dem folglich eine Vergrößerung eines Bildes des Auges veränderbar ist.

Die EP 2 016 888 A1 beschreibt ein ophthalmologisches Analysegerät zur Topographiemessung, welches im Wesentlichen eine kegelförmige Placidoscheibe aufweist und über eine Kamera zur Aufnahme eines projizierten Bildmusters auf einem Auge verfügt. Die Placidoscheibe ist eine Blendeneinrichtung, die mit einer Beleuchtungseinrichtung, d. h. einer Hintergrundbeleuchtung versehen ist. Weiter sind im Zentrum der Placidoscheibe Infrarot-Leuchtdioden angeordnet.

Die US 5,159,361 A1 betrifft im ein Topografiesystem, wobei ein Objektiv eine variable Vergrößerung einer Hornhaut ermöglicht.

Der vorliegenden Erfindung liegt daher die Aufgabe zu Grunde, ein ophthalmologisches Analysegerät sowie ein Analyseverfahren mit einem ophthalmologischen Analysegerät vorzuschlagen, mit dem die Untersuchungsmöglichkeiten eines Topografiesystems erweitert und verbessert werden.

Diese Aufgabe wird durch eine Vorrichtung mit den Merkmalen des Anspruchs 1 und ein Verfahren mit den Merkmalen des Anspruchs 5 gelöst.

Das erfindungsgemäße, ophthalmologische Analysegerät, insbesondere zur Messung einer Topografie einer Oberfläche eines Auges, weist eine Projektionsvorrichtung und eine Beobachtungsvorrichtung auf, wobei die Projektionsvorrichtung zumindest eine Beleuchtungseinrichtung und eine Blendeneinrichtung umfasst, wobei die Beleuchtungseinrichtung zumindest eine erste Lichtquelle aufweist, wobei mittels der Blendeneinrichtung ein Bildmuster auf einer Oberfläche eines Auges abbildbar ist, wobei die Beobachtungsvorrichtung eine Kamera und ein Objektiv aufweist, wobei mittels der Beobachtungsvorrichtung Bilder des abgebildeten Bildmusters aufgenommen werden können, wobei aus den Bildern eine Topografie der Oberfläche abgeleitet werden kann, wobei mittels des Objektivs eine Vergrößerung des Bildes veränderbar ist.

Dadurch, dass die Beobachtungsvorrichtung eine Kamera und ein Objektiv aufweist, sind die erhaltenen Bilder qualitativ gut verwertbar. So wird es möglich, je nach Art der Messung eine Vergrößerung des Objektivs so zu wählen, dass das zu messende Objekt bzw. die zu messende Eigenschaft so visuell darstellbar ist, dass überhaupt eine Auswertung des Bildes sowie besonders genaue Messergebnisse möglich werden.

Bei einer sehr großen Vergrößerung ist es möglich, den Tränenfilm so zu fokussieren, dass im Tränenfilm befindliche Partikel, wie zum Beispiel Staubpartikel oder Fremdkörper, sichtbar werden. Eine mögliche Bewegung dieser Partikel kann so nachverfolgt und hinsichtlich Bewegungsrichtung und Geschwindigkeit gemessen werden. Daraus kann abgeleitet werden, in welche Richtung bzw. mit welcher Geschwindigkeit der Tränenfilm fließt. Diese Messung kann dazu dienen eine Tränenfilmqualität noch genauer zu bestimmen.

Das Objektiv kann dazu einen Vergrößerungswechsler aufweisen, mittels dem zumindest eine Linse in einen Strahlengang des Objektivs einbringbar bzw. aus diesem entfernbar ist. Gegenüber einer variablen Vergrößerungseinstellung, welche ebenfalls denkbar ist, ist ein Vergrößerungswechsler besonders einfach und kostengünstig herstellbar. Auch wird es dann möglich immer gleichbleibende, standardisierte Vergrößerungen für die verschiedenen Messungen zu verwenden, was die Messungen sowie eine Auswertung bzw. Analyse der Bilder erheblich vereinfacht.

Mittels des Objektivs können nach einer nicht der Erfindung zugehörigen Ausführungsform vorteilhaft zumindest drei Vergrößerungen ausbildbar sein. Eine erste, normale Vergrößerung kann zur Messung der Topografie der Oberfläche des Auges verwendet werden. Eine zweite, große Vergrößerung, kann zur Messung bzw. Analyse eines Tränenfilms verwendet werden. Dabei kann insbesondere bei geringer Tiefenschärfe beispielsweise auf eine Lipidschicht fokussiert werden, um deren Dicke zu bestimmen. Eine dritte, kleine Vergrößerung kann insbesondere für meibometrische Untersuchungen genutzt werden, da eine Bildaufnahme von Meibomdrüsen unter anderem einen vergrößerten Abstand des Analysegeräts relativ zum Auge erfordert.

Auch kann die erste Lichtquelle Licht in einem überwiegend monochromatischen Spektrum emittieren. So kann das Licht der ersten Lichtquelle besser von der Beobachtungsvorrichtung erkannt werden.

Vorteilhaft kann die erste Lichtquelle Licht in einem überwiegend infrarotem Spektrum emittieren. Infrarotes Licht hat eine besonders geringe Blendwirkung auf das zu untersuchende Auge und ist einfach zur Verfügung zu stellen.

Die Beleuchtungseinrichtung kann nach einer nicht der Erfindung zugehörigen Ausführungsform zumindest eine weitere Lichtquelle aufweisen, die polychromatisches Licht in einem überwiegend sichtbaren Spektrum emittieren kann. Neben der Verwendung von im wesentlichen monochromatischen Licht bzw. Licht eines vergleichsweise schmalen Wellenlängenbereichs kann die weitere Lichtquelle eine Beleuchtung der Oberfläche des Auges mit polychromatischen, sichtbaren Licht ermöglichen, wodurch zusätzliche Messungen von Eigenschaften des Auges und eine Erzielung genauerer Messergebnisse möglich werden. Gegenüber monochromatischen oder infraroten Licht wird es dann möglich mit polychromatischen Licht einen Rötungsgrad des Auges zu bestimmen. Auch wird es möglich, eine Dicke eines Tränenfilms oder einer Lipidschicht des Tränenfilms vergleichsweise genau nicht invasiv zu messen, da mit polychromatischen Licht farbige Interferenzmuster der Lipidschicht erzeugt werden können, die für derartige Messungen verwendbar sind. Besonders vorteilhaft ist es, wenn nach einer nicht der Erfindung zugehörigen Ausführungsform die weitere Lichtquelle überwiegend weißes Licht emittieren kann. Mit weißem Licht kann eine besonders gute Farbwiedergabe erzielt werden, was insbesondere bei einer Messung des Rötungsgrades des Auges und der Erzeugung farbiger Interferenzmuster eine Messgenauigkeit wesentlich verbessert.

Nach einer nicht der Erfindung zugehörigen Ausführungsform kann die weitere Lichtquelle aus einer Vielzahl gleichmäßig verteilter Leuchtdioden zusammengesetzt sein. Beispielsweise können die Leuchtdioden selbst das Bildmuster ausbilden. So können die Leuchtdioden ringförmig angeordnet sein oder auch hinter einer Blende, die das Bildmuster auf das Auge projizieren kann. Die Leuchtdioden, die die weitere Lichtquelle ausbilden, können dabei zusammen mit Leuchtdioden, die die erste Lichtquelle ausbilden, an der Beleuchtungseinrichtung angeordnet sein. So wird es möglich, die betreffenden Leuchtdioden nach Bedarf, getrennt voneinander oder zusammen zu betreiben. Eine Anzahl der Leuchtdioden der ersten Lichtquelle kann dabei zu einer Anzahl der Leuchtdioden der weiteren Lichtquelle in einem Verhältnis von 1 zu 3 gewählt sein.

Besonders vorteilhaft ist es, wenn nach einer nicht der Erfindung zugehörigen Ausführungsform in einer Richtung einer optischen Achse des Auges ausrichtbaren Geräteachse der Blendeneinrichtung ein Durchlass in der Blendeneinrichtung für einen Strahlengang der Beobachtungsvorrichtung ausgebildet ist. So kann die Oberfläche des Auges durch die Blendeneinrichtung allseitig mit dem Bildmuster beleuchtet werden. Die Blendeneinrichtung kann so beispielsweise aus einem leuchtenden Ring oder einer Mehrzahl derartiger, konzentrischer Ringe zur Erzeugung eines Placidobildmusters ausgebildet sein. Wenn die Blendeneinrichtung nach der optischen Achse des Auges mit ihrer Geräteachse ausgerichtet ist, kann der Strahlengang der Beobachtungsvorrichtung unmittelbar entlang der optischen Achse oder auf dieser verlaufen, sodass eine frontale Beobachtung des Auges durch den Durchlass hindurch ermöglicht und dann insbesondere pupillometrische Messungen wesentlich vereinfacht.

So kann das Analysegerät nach einer nicht der Erfindung zugehörigen Ausführungsform auch eine Blendvorrichtung zur Erzeugung eines Blendreizes auf das Auge aufweisen, wobei die Blendvorrichtung eine Blendlichtquelle und einen Strahlenteiler zur Einspiegelung der Blendlichtquelle in den Strahlengang der Beobachtungsvorrichtung aufweisen kann. So kann das Auge mit dieser Blendvorrichtung geblendet werden, wobei gleichzeitig eine Reaktion des Auges mittels der Beobachtungsvorrichtung aufgenommen werden kann. Zum Beispiel kann eine durch die Blendung veranlasste Bewegung der Pupille erfasst und ausgewertet werden. Die Blendvorrichtung kann dabei unabhängig von der Beleuchtungseinrichtung bzw. von mit dieser erzeugten Bildmustern betrieben werden.

Weiter ist es möglich, die Beleuchtungseinrichtung und/oder die Blendvorrichtung zur Blendung des Auges mit dem Ziel zu nutzen, dass eine vermehrte Produktion von Tränenflüssigkeit erfolgt. So kann die Bildung eines Tränenfilms überprüft bzw. gemessen werden.

Das erfindungsgemäße Analyseverfahren wird mit einem ophthalmologischen Analysegerät zur Messung einer Topografie einer Oberfläche eines Auges durchgeführt, wobei das Analysegerät eine Projektionsvorrichtung und eine Beobachtungsvorrichtung umfasst, wobei die Projektionsvorrichtung zumindest eine Beleuchtungseinrichtung und eine Blendeneinrichtung umfasst, wobei mittels der Blendeneinrichtung ein Bildmuster auf die Oberfläche des Auges abgebildet wird, wobei mittels der Beobachtungsvorrichtung Bilder des abgebildeten Bildmusters aufgenommen werden, wobei aus den Bildern ein Tränenfilmfluss abgeleitet wird, wobei eine Bewegungsgeschwindigkeit von auf der Oberfläche befindlichen Partikeln gemessen wird.

Bei einer sehr großen Vergrößerung ist es möglich, den Tränenfilm so zu fokussieren, dass im Tränenfilm befindliche Partikel, wie zum Beispiel Staubpartikel oder Fremdkörper, sichtbar werden. Eine mögliche Bewegung dieser Partikel wird dann nachverfolgt und hinsichtlich Bewegungsrichtung und Geschwindigkeit gemessen. Daraus kann abgeleitet werden, in welche Richtung bzw. mit welcher Geschwindigkeit der Tränenfilm fließt. Diese Messung dient dazu eine Tränenfilmqualität noch genauer zu bestimmen.

In einer Ausführungsform des Verfahrens kann das Analysegerät eine Auswertevorrichtung aufweisen, mittels der die Bilder analysiert werden. Vorteilhaft kann die Auswertevorrichtung im Analysegerät selbst angeordnet sein und eine Verarbeitung der Bilder sowie eine visualisierte Ausgabe der von der Auswertevorrichtung ermittelten Messergebnisse ermöglichen. Die Auswertevorrichtung kann insbesondere Mittel zur Datenverarbeitung umfassen, die dann auch eine digitale Verarbeitung der Bilder durchführen können. Auch ist es denkbar, dass die Mittel zur Datenverarbeitung Datenspeicher mit einer Datenbank aufweisen, wobei die Datenbank Vergleichsdatensätze von Bildern oder Messparametern aufweisen kann. Aus derartigen Vergleichsdatensätzen können dann, beispielsweise durch einen Bildvergleich, vereinfachte Rückschlüsse auf wahrscheinliche Messergebnisse oder auch Korrekturen von Messergebnissen durchgeführt werden. Eine Auswertung kann dadurch wesentlich beschleunigt und eine Messgenauigkeit noch weiter erhöht werden. Auch kann die Beleuchtungseinrichtung zumindest eine Lichtquelle aufweisen, die polychromatisches Licht in einem überwiegend sichtbaren Spektrum emittiert, wobei dann aus den Bildern ein Tränenfilm auf der Oberfläche bestimmt werden kann. Insbesondere durch die Verwendung von polychromatischen, sichtbaren Licht zur Beleuchtung des Auges bzw. des Tränenfilms mit dem Bildmuster wird eine besonders genaue Untersuchung des Tränenfilms möglich.

Weiter kann aus den Bildern ein Rötungsgrad des Auges bestimmt werden, wobei ein Rotanteil in einem Bereich des Auges gemessen werden kann. Eine derartige Messung kann nicht mit Infrarotlicht oder monochromatischen Licht aufgrund der mangelnden Farbwiedergabe durchgeführt werden. So kann bei der Messung eine Rötung des die Iris des Auges umgebenden Bereichs durch Quantifizierung der roten Bildanteile aufgrund der guten Farbwiedergabe des polychromatischen, sichtbaren Lichts messtechnisch ermittelt werden. Die Messung kann als eine vergleichende Messung durchgeführt werden, beispielsweise durch einen Vergleich mit einem Referenzbild.

Auch kann aus den Bildern eine Tränenfilmaufreißzeit abgeleitet werden, wobei eine Veränderung des Tränenfilms gemessen werden kann. Die Messung einer Tränenfilmaufreißzeit ist bei einer normalen Vergrößerung möglich und kann bei einer Beleuchtung mit Infrarotlicht oder sichtbaren Licht durchgeführt werden. Die auf die Oberfläche des Auges projizierten Bildmuster, wie zum Beispiel Placidoringe, ermöglichen eine Erkennung eines Aufreißens des Tränenfilms, insbesondere durch eine Veränderung des betreffenden Bildmusters. Eine Aufreißzeit eines Tränenfilms kann als wesentlicher Parameter zur Qualitätsbestimmung des Tränenfilms angesehen werden.

Weiter kann aus den Bildern eine Lipidschicht auf dem Tränenfilm bestimmt werden, wobei die Lipidschicht durch Interferenzfarben gemessen werden kann. Eine Lipidschicht betrifft eine äußere Schicht des Tränenfilms, wobei nach der Lipidschicht eine mittlere, wässrige Schicht und eine an die Hornhaut angrenzende, innere Schicht (Muzinschicht) folgt. Die Lipidschicht ist etwa 100 nm dick, verhindert ein schnelles Verdunsten der wässrigen Schicht und bildet sich aus einem Sekret der Meibomdrüsen. Da es sich bei der Lipdschicht um eine sehr dünne Schicht des Tränenfilms handelt, kann diese sehr einfach anhand von Interferenzfarben gemessen werden. So kann das Auge bzw. der Tränenfilm mit polychromatischen Licht beleuchtet werden, wodurch sich anhand der vorgenannten Schicht die Dicke der Interferenzfarben bzw. Interferenzmuster sichtbar auf dem Tränenfilm ergeben. Mögliche Tränenfilmeigenschaften werden so noch genauer bestimmbar.

Dadurch wird es möglich, eine Lipidschichtdicke bzw. Dickeverteilung der Lipidschicht sowie deren Dicke zu bestimmen als auch eine Funktion der Meibomdrüsen anhand der messbaren Menge an Lipiden zu untersuchen. Vorzugsweise kann für eine derartige Messung eine große Vergrößerung gewählt werden.

Weitere vorteilhafte Ausführungsformen des Verfahrens ergeben sich aus den Merkmalsbeschreibungen der aus dem Vorrichtungsanspruch 1 rückbezogenen Unteransprüche.

Nachfolgend wird eine bevorzugte Ausführungsform der Erfindung unter Bezugnahme auf die beigefügten Zeichnungen näher erläutert.

Es zeigen:
- **Fig. 1:**: Eine vereinfachte, schematische Schnittansicht einer Ausführungsform eines Analysegerätes;
- **Fig. 2:**: eine Vorderansicht des Analysegerätes.

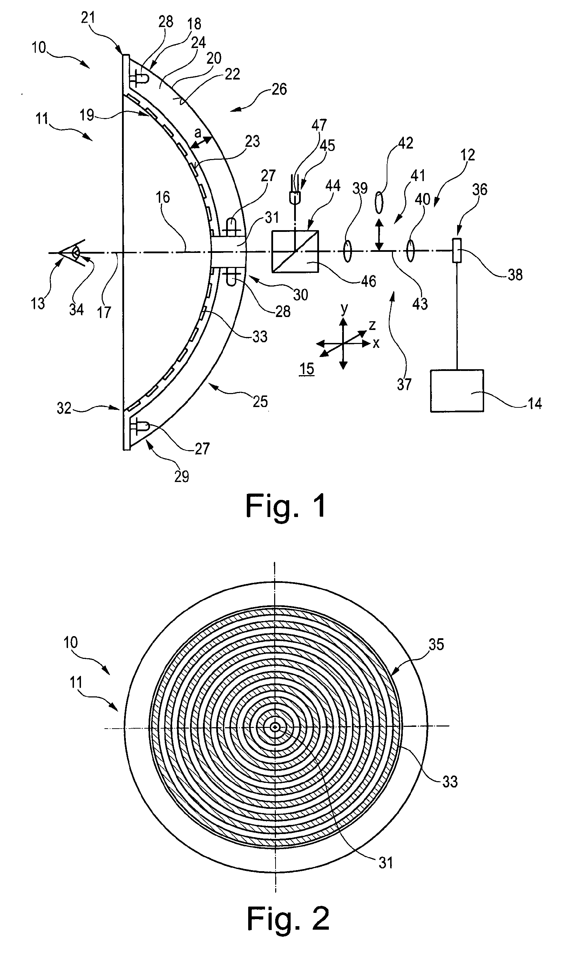

Eine Zusammenschau der **Fig. 1** und **2** zeigt eine Ausführungsform eines Analysegerätes 10, welches im Wesentlichen aus einer Projektionsvorrichtung 11 und einer Beobachtungsvorrichtung 12 zur Beobachtung eines Auges 13 gebildet ist. Weiter umfasst das Analysegerät 10 eine Auswertevorrichtung 14 mit hier nicht näher dargestellten Mitteln zur Datenverarbeitung und -ausgabe sowie eine Positioniervorrichtung 15 zur Positionierung des Analysegerätes 10 relativ zu dem Auge 13 in drei Raumrichtungen mit den senkrecht aufeinander stehenden Richtungskomponenten x, y, z, wie hier symbolisch angedeutet. Das Analysegerät wird dabei so positioniert, dass eine Geräteachse 16 des Analysegerätes 10 mit einer optischen Achse 17 des Auges 13 in Übereinstimmung gelangt.

Die Projektionsvorrichtung 11 ist als ein Hohlkugelsegment 18 ausgebildet und umfasst eine Rasterblende 19 und einen Reflektor 20, welche in einem schematisch angedeuteten Gehäuse 21 so angebracht sind, dass der Reflektor 20 mit einer reflektierenden Fläche 22 zu einer Oberfläche 23 des Reflektors 20 stets den gleichen Abstand a aufweist, und so ein gekrümmter Blendenraum 24 gebildet wird. Die Oberfläche 23 des Reflektors 20 ist stark reflektierend, so dass der Blendenraum 24 gleichmäßig lichterfüllt ist, wenn eine erste Lichtquelle 25 oder eine weitere Lichtquelle 26 eingeschaltet ist. Die Lichtquellen 25 und 26 sind jeweils aus Leuchtdioden 27 bzw. 28 gebildet, welche jeweils in einer gleichmäßig verteilten Vielzahl in Art eines Rings 29 an einem Umfang der Rasterblende 19 und in Art eines Rings 30 an einem Durchlass 31 der Rasterblende 19 angeordnet sind. Die Rasterblende 19 ist im Wesentlichen aus einem transparenten Korpus 32 mit kreisringförmigen Blendenelementen 33 gebildet, welche auftreffendes Licht aus einer Lichtquelle 25 und/oder 26 in den Blendenraum 24 reflektieren. Somit wird auf einer Hornhaut 34 des Auges 13 das in Fig. 2 ersichtliche Bildmuster 35 abgebildet, wobei das Bildmuster 35 mittels der Beobachtungsvorrichtung 12 aufgenommen wird.

Die Beobachtungsvorrichtung 12 weist eine Kamera 36 mit einem Objektiv 37 auf, wobei die Kamera 36 unter anderem einen optischen Videosensor 38 umfasst, der unmittelbar mit der Auswertevorrichtung 14 verbunden ist. Das Objektiv 37 ist aus zwei Linsen 39 und 40 gebildet, wobei ergänzend, wie hier mit dem Doppelpfeil angedeutet, ein Vergrößerungswechsler 41 vorgesehen ist, mittels dem weitere Linsen 42 in einen Strahlengang 43 der Beobachtungsvorrichtung 12 eingeschwenkt werden können. Somit wird es möglich das Auge 13 in zumindest drei unterschiedlichen Vergrößerungen aufzunehmen und zu betrachten. In dem Strahlengang 43 ist zwischen der Projektionsvorrichtung 11 und dem Objektiv 37 ein Strahlenteiler 44 einer Blendvorrichtung 45 angeordnet, welcher aus einem Prismensystem 46 besteht, jedoch auch aus teildurchlässigen ebenen Spiegeln zusammengesetzt sein kann. Über dem Strahlenteiler 44 kann eine Blendlichtquelle 47 in dem Auge 13 abgebildet werden. Auf diese Weise kann man ganz unabhängig von der Projektionsvorrichtung 11 einen Blendreiz auf dem Auge 13 erzeugen, sodass dessen Reaktion mit Hilfe der ohnehin verfügbaren Beobachtungsvorrichtung 12 erfasst und von der Auswertevorrichtung 14 auch numerisch bestimmt werden kann. Ein spezielles Gerät ist für eine solche Messung dementsprechend nicht mehr erforderlich.

Hinsichtlich der Arbeitsweise des Analysegeräts 10 ist vorgesehen, dass die Leuchtdioden 27 Licht in einem überwiegend infraroten Spektrum und die Leuchtdioden 28 polychromatisches, weißes Licht in einem überwiegend sichtbaren Spektrum emittieren bzw. abstrahlen können. Die erste Lichtquelle 25 wird von circa 50 Leuchtdioden 27 und die weitere Lichtquelle 26 von circa 150 Leuchtdioden 28 gebildet. Innerhalb einer Messung kann nach Bedarf die erste Lichtquelle 25 oder die weitere Lichtquelle 26 zur Beleuchtung des Auges 13 eingeschaltet werden. Insbesondere für eine Bestimmung einer Topografie des Auges 13 ist es ausreichend, die erste Lichtquelle 25 bei einer normalen Vergrößerung des Objektivs 37 zu verwenden. Die erste Lichtquelle 25 wird auch für meibometrische Untersuchungen eingesetzt, wobei hier eine kleine Vergrößerung des Objektivs 37 gewählt wird und eine Vergrößerung eines Abstands des Analysegerätes 10 zu dem Auge 13 vorgesehen ist. Die weitere Lichtquelle 26 wird für eine Analyse eines Tränenfilms, insbesondere einer Lipidschicht des Tränenfilms eingesetzt, wobei dann eine besonders große Vergrößerung des Objektivs 37 gewählt wird. Im Übrigen wird die Blendlichtquelle 47 für pupillometrische Messungen eingesetzt.

## Patentansprüche

1. Ophthalmologisches Analysegerät (10), insbesondere zur Messung einer Topografie einer Oberfläche eines Auges, mit einer Projektionsvorrichtung (11) und einer Beobachtungsvorrichtung (12), wobei die Projektionsvorrichtung zumindest eine Beleuchtungseinrichtung und eine Blendeneinrichtung (19) umfasst, wobei die Beleuchtungseinrichtung zumindest eine erste Lichtquelle (25) aufweist, wobei mittels der Blendeneinrichtung ein Bildmuster (35) auf einer Oberfläche eines Auges (13) abbildbar ist, wobei die Beobachtungsvorrichtung eine Kamera (36) und ein Objektiv (37) aufweist, wobei mittels der Beobachtungsvorrichtung Bilder des abgebildeten Bildmusters aufgenommen werden können, wobei aus den Bildern eine Topografie der Oberfläche abgeleitet werden kann,
**dadurch gekennzeichnet,**
**dass** mittels des Objektivs eine Vergrößerung des Bildes derart veränderbar ist, dass sich im Tränenfilm befindliche Partikel bei einer großen Vergrößerung sichtbar werden.

2. Analysegerät nach Anspruch 1,
**dadurch gekennzeichnet,**
**dass** das Objektiv (37) einen Vergrößerungswechsler (41) aufweist, mittels dem zumindest eine Linse (42) in einen Strahlengang (43) des Objektivs einbringbar bzw. aus diesem entfernbar ist.

3. Analysegerät nach Anspruch 1 oder 2,
**dadurch gekennzeichnet,**
**dass** die erste Lichtquelle (25) Licht in einem überwiegend monochromatischen Spektrum emittieren kann.

4. Analysegerät nach Anspruch 3,
**dadurch gekennzeichnet,**
**dass** die erste Lichtquelle (25) Licht in einem überwiegend infraroten Spektrum emittieren kann.

5. Analyseverfahren mit einem ophthalmologischen Analysegerät (10) zur Messung einer Topografie einer Oberfläche eines Auges, mit einer Projektionsvorrichtung (11) und einer Beobachtungsvorrichtung (12), wobei die Projektionsvorrichtung zumindest eine Beleuchtungseinrichtung und eine Blendeneinrichtung (19) umfasst, wobei mittels der Blendeneinrichtung ein Bildmuster (35) auf die Oberfläche des Auges (13) abgebildet wird, wobei mittels der Beobachtungsvorrichtung Bilder des abgebildeten Bildmusters aufgenommen werden,
**dadurch gekennzeichnet,**
**dass** aus den Bildern ein Tränenfilmfluss abgeleitet wird, wobei eine Bewegungsgeschwindigkeit von sich auf der Oberfläche befindlichen Partikeln gemessen wird.

6. Analyseverfahren nach Anspruch 5,
**dadurch gekennzeichnet,**
**dass** das Analysegerät (10) eine Auswertevorrichtung (14) aufweist, mittels der die Bilder analysiert werden.

7. Analyseverfahren nach Anspruch 5 oder 6,
**dadurch gekennzeichnet,**
**dass** die Beleuchtungseinrichtung zumindest eine Lichtquelle (26) aufweist, die polychromatisches Licht in einem überwiegend sichtbaren Spektrum emittiert, wobei aus den Bildern ein Tränenfilm auf der Oberfläche bestimmt wird.

8. Analyseverfahren nach Anspruch 7,
**dadurch gekennzeichnet,**
**dass** aus den Bildern ein Rötungsgrad des Auges (13) bestimmt wird, wobei ein Rotanteil in einem Bereich des Auges gemessen wird.

9. Analyseverfahren nach einem der Ansprüche 5 bis 8,
**dadurch gekennzeichnet,**
**dass** aus den Bildern eine Tränenfilmaufreißzeit abgeleitet wird, wobei eine Veränderung des Tränenfilms gemessen wird.

10. Analyseverfahren nach einem der Ansprüche 5 bis 9,
**dadurch gekennzeichnet,**
**dass** aus den Bildern eine Lipidschicht auf dem Tränenfilm bestimmt wird, wobei die Lipidschicht durch Interferenzfarben gemessen wird.

11. Analyseverfahren nach Anspruch 10,
**dadurch gekennzeichnet,**
**dass** eine Lipidschichtdicke gemessen wird.

## Claims

1. An ophthalmological analysis instrument (10), in particular for measuring a topography of a surface of an eye, having a projection apparatus (11) and a monitoring apparatus (12), the projection apparatus comprising at least one illumination device and an aperture device (19), the illumination device having at least one first light source (25), it being possible to image an image pattern (35) on a surface of an eye (13) by means of the aperture device, the monitoring apparatus having a camera (36) and an objective lens (37), images of the imaged image pattern being recordable by means of the monitoring apparatus, and a topography of the surface being derivable from the images,
**characterized in that**
a magnification of the image can be varied by means of the objective lens in such a manner that particles situated in the tear film become visible at high magnification.

2. The analysis instrument according to claim 1,
**characterized in that**
the objective lens (37) has a magnification changer (41), by means of which the at least one lens (42) can be introduced into a beam path (43) of the objective lens and removed therefrom.

3. The analysis instrument according to claim 1 or 2,
**characterized in that**
the first light source (25) can emit light in a predominantly monochromatic spectrum.

4. The analysis instrument according to claim 3,
**characterized in that**
the first light source (25) can emit light in a predominantly infrared spectrum.

5. An analysis method using an ophthalmological analysis instrument (10) for measuring a topography of a surface of an eye, said analysis instrument having a projection apparatus (11) and a monitoring apparatus (12), the projection apparatus comprising at least one illumination device and an aperture device (19), it being possible to image an image pattern (35) onto the surface of the eye (13) by means of the aperture device, images of the imaged image pattern being recorded by means of the monitoring apparatus,
**characterized in that**
a tear film flow is derived from the images, a speed of movement of particles situated on the surface being measured.

6. The analysis method according to claim 5,
**characterized in that**
the analysis instrument (10) has an evaluation apparatus (14), by means of which the images are analyzed.

7. The analysis method according to claim 5 or 6,
**characterized in that**
the illumination device has at least one light source (26) that emits polychromatic light in a predominantly visible spectrum, a tear film on the surface being determined from the images.

8. The analysis method according to claim 7,
**characterized in that**
a degree of reddening of the eye (13) is determined from the images, a proportion of red in a region of the eye being measured.

9. The analysis method according to one of claims 5 to 8,
**characterized in that**
a tear film break-up time is derived from the images, a change to the tear film being measured.

10. The analysis method according to one of claims 5 to 9,
**characterized in that**
a lipid layer on the tear film is determined from the images, the lipid layer being measured by interference colors.

11. The analysis method according to claim 10,
**characterized in that**
a lipid layer thickness is measured.

## Revendications

1. Appareil d'analyse ophtalmologique (10), notamment pour mesurer une topographie d'une surface d'un oeil, comportant un dispositif de projection (11) et un dispositif d'observation (12), le dispositif de projection comprenant au moins un moyen d'éclairage et un moyen de diaphragme (19), le moyen d'éclairage comportant au moins une première source lumineuse (25), un motif d'images (35) pouvant être imagé sur une surface d'un oeil (13) par le moyen de diaphragme, le dispositif d'observation comportant une caméra (36) et un objectif (37), des images du motif d'images imagé pouvant être prises par le dispositif d'observation et une topographie de la surface pouvant être dérivée à partir des images,
**caractérisé en ce qu'**
un grossissement de l'image est changeable par l'objectif de telle manière que des particules se trouvant dans le film lacrymal deviennent visibles à un forte grossissement.

2. Appareil d'analyse selon la revendication 1,
**caractérisé en ce que**
l'objectif (37) comporte un changeur de grossissement (41), au moyen duquel au moins une lentille (42) peut être introduite dans un trajet de faisceau (43) de l'objectif et retirée de celui-ci.

3. Appareil d'analyse selon la revendication 1 ou 2,
**caractérisé en ce que**
la première source lumineuse (25) peut émettre de la lumière dans un spectre principalement monochromatique.

4. Appareil d'analyse selon la revendication 3,
**caractérisé en ce que**
la première source lumineuse (25) peut émettre de la lumière dans un spectre principalement infrarouge.

5. Procédé d'analyse utilisant un appareil d'analyse ophtalmologique (10) pour mesurer une topographie d'une surface d'un oeil, l'appareil comportant un dispositif de projection (11) et un dispositif d'observation (12), le dispositif de projection comprenant au moins un moyen d'éclairage et un moyen de diaphragme (19), un motif d'images (35) étant imagé sur la surface de l'oeil (13) par le moyen de diaphragme, des images du motif d'images imagé étant prises par le dispositif d'observation,
**caractérisé en ce qu'**
un écoulement du film lacrymal est dérivé à partir des images, une vitesse de mouvement des particules se trouvant sur la surface étant mesurée.

6. Procédé d'analyse selon la revendication 5,
**caractérisé en ce que**
l'appareil d'analyse (10) comporte un dispositif d'évaluation (14), par lequel les images sont analysées.

7. Procédé d'analyse selon la revendication 5 ou 6,
**caractérisé en ce que**
le moyen d'éclairage comporte au moins une source lumineuse (26) qui émet de la lumière polychromatique dans un spectre principalement visible, un film lacrymal sur la surface étant déterminé à partir des images.

8. Procédé d'analyse selon la revendication 7,
**caractérisé en ce qu'**
un degré de rougeur de l'oeil (13) est déterminé à partir des images, une part rouge dans une région de l'oeil étant mesurée.

9. Procédé d'analyse selon l'une quelconque des revendications 5 à 8,
**caractérisé en ce qu'**
un temps de rupture du film lacrymal est dérivé à partir des images, un changement du film lacrymal étant mesuré.

10. Procédé d'analyse selon l'une quelconque des revendications 5 à 9,
**caractérisé en ce qu'**
une couche lipidique sur le film lacrymal est déterminée à partir des images, la couche lipidique étant mesurée par l'intermédiaire des couleurs d'interférence.

11. Procédé d'analyse selon la revendication 10,
**caractérisé en ce qu'**
une épaisseur de la couche lipidique est mesurée.
